# EUROPÄISCHE PATENTSCHRIFT

(11) **EP 2 025 296 B1**
(45) Veröffentlichungstag und Bekanntmachung des Hinweises auf die Patenterteilung: **13.11.2013**
(21) Anmeldenummer: 08161406.7
(22) Anmeldetag: 30.07.2008
(51) Int. Cl.: A61B 17/88, B25B 23/143, A61C 8/00

(54) **Drehmomentschlüssel, insbesondere für den medizinischen Bereich**
Torque wrench, in particular for medical applications
Clé dynamométrique, en particulier pour le domaine médical

(30) Priorität: 30.07.2007 DE 202007010665 U
(43) Veröffentlichungstag der Anmeldung: 18.02.2009
(73) Patentinhaber: Zrinski AG, 78573 Wurmlingen (DE)
(72) Erfinder: Reitzig, Cliff-Georg, 78604 Weilheim-Rietheim (DE)
(74) Vertreter: Heisel, Wolfgang

(56) Entgegenhaltungen:
- EP-A- 1 880 802
- DE-A1- 3 149 561
- DE-U1- 8 912 570
- US-A- 3 753 625
- US-B1- 7 296 500

## Beschreibung

### Technisches Gebiet

Die Erfindung bezieht sich auf eine Vorrichtung zum Eindrehen von Schrauben und dgl., insbesondere von im medizinischen Bereich verwendeten Knochenschrauben, die ein Adapterelement zur Aufnahme eines Handgriffes oder eines Hilfswerkzeugs und ein Aufnahmeelement für die Schrauben aufweist, während das Adapterelement und das Aufnahmeelement miteinander über ein Drehmomentbegrenzungsmittel mit einem Abscherstift zum Begrenzen eines beim Eindrehen der Schrauben aufzubringendes maximal zulässiges Drehmoments miteinander über ein erstes und zweites jeweils scheibenförmig ausgebildetes und mit jeweils einer Gleitfläche versehenes Gleitflächenelement gekoppelt und koaxial zueinander geführt sind, und zwar indem diese Gleitflächenelemente jeweils eine ortsfeste Ausnehmung für die gemeinsame Aufnahme des Abscherstiftes aufweisen, wobei das erste Gleitflächenelement dem Adapterelement und das zweite Gleitflächenelement dem Aufnahmeelement zugeordnet ist.

### Definitionen

In der nachfolgenden Beschreibung werden unterschiedliche Begriffe verwendet, denen folgende Bedeutungen zugewiesen werden.

Unter dem Begriff Aufnahmeelement ist eine Vorrichtung zu verstehen, die dazu geeignet ist, eine Schraube oder Mutter aufzunehmen, um diese in eine Drehbewegung zu versetzen. So kann beispielsweise als Aufnahmeelement ein so genanntes Schraubenbit oder aber auch ein Schraubendreher und eine Imbuseinrichtung verstanden werden. Eine Imbuseinrichtung ist derart ausgestaltet, dass sie dazu geeignet ist, in eine Imbusausnehmung einer Schraube einzugreifen, um diese in eine entsprechende Drehung zu versetzen. Die Drehung selbst wird dadurch ausgeübt, dass eine form- und kraftschlüssige Verbindung zwischen dem Aufnahmeelement und dem Gegenstand, der in Drehbewegung zu versetzen ist, hergestellt wird. Eine einfache Ausführung eines Aufnahmeelements ist ein Schraubendreher. Er weist an seinem freien Ende einen schmalen Steg oder eine kreuzartige Ausbildung auf, womit dieser in entsprechende Ausnehmungen einer Schraube eintauchen kann. Durch eine Drehbewegung kann dann die Schraube damit in eine Drehbewegung versetzt werden.

Unter dem Begriff Adapterelement wird ein Element verstanden, das dazu geeignet ist, ein Betätigungsmittel, wie beispielsweise einen Handgriff aufzunehmen. Dieser Handgriff kann aufsteckbar sein, er kann jedoch auch mit dem Adapterelement einstückig ausgebildet sein. Als Alternative zu einem Handgriff kann auch jegliches Hilfswerkzeug vorgesehen sein, das dazu geeignet ist, mit dem Adapterelement gekoppelt zu werden. So kann beispielsweise vorgesehen sein, das Adapterelement mit einem elektrischen Schraubendreher zu verbinden.

Vorzugsweise sind das Aufnahmeelement und das Adapterelement zylinderförmig und rotationssymmetrisch ausgebildet. Insbesondere durch die Anwendung eines elektrischen Schraubendrehers wird das Adapterelement in Rotation versetzt.

Zur Begrenzung des über das Adapterelement aufgebrachten Drehmoments, ist ein so genanntes Drehmomentbegrenzungsmittel vorgesehen. Es begrenzt die Übertragung des aufgebrachten Drehmoments über das Adapterelement in Bezug auf das Aufnahmeelement. Vorzugsweise ist unmittelbar zwischen dem Aufnahmeelement und dem Adapterelement das Drehmomentbegrenzungsmittel angeordnet. Übersteigt das Drehmoment, das über das Adapterelement aufgebracht wird, das zulässige und zuvor ausgewählte Drehmoment, so sieht dieses Drehbegrenzungsmittel vor, dass die Kräfte nicht weiter auf das Aufnahmeelement übertragen werden. Somit ist gewährleistet, dass das zuvor eingestellte Drehmoment mit seinem maximalen Betrag auf das Aufnahmeelement übertragen wird.

Das Drehmomentbegrenzungsmittel kann aus einem Bauteil bestehen. Alternativ ist vorgesehen, dass das Drehmomentbegrenzungsmittel mehrere Bauteile umfasst. So ist das freie Ende des Adapterelements mit einer Gleitfläche versehen, die auf einer weiteren Gleitfläche gleitet, die an der zu dem Adapterelement zugewandten Seite des Aufnahmeelements vorgesehen ist. Ein Abscherstift stellt zunächst die starre Kopplung zwischen den beiden Gleitflächen her.

Die Erfindung, die nachfolgend beschrieben wird, ist nicht beschränkt auf die Anwendung im medizinischen Bereich. Daher kann die Erfindung beispielsweise auch im Kraftfahrzeugbereich, in der Luftfahrtindustrie oder aber auch in der Fein- und Mikromechanik angewendet werden.

### Stand der Technik

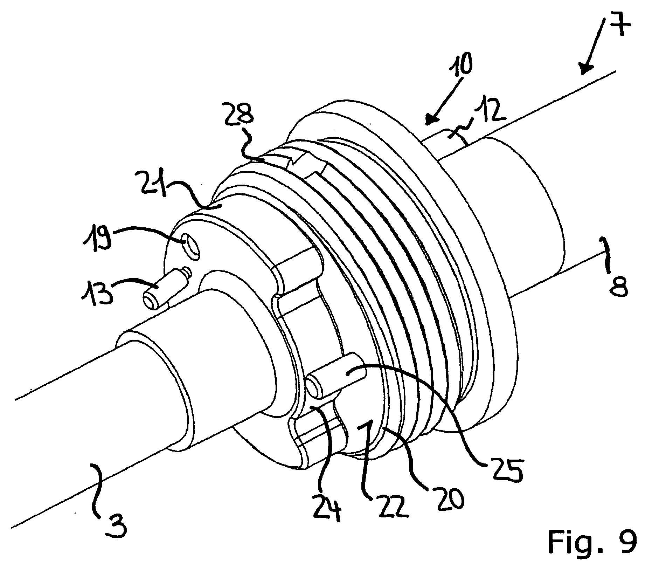

In diesem Zusammenhang ist aus der US 3753625 (FABRIZIO ET AL) 21.08.1973 ein Halter für Schneidwerkzeuge bekannt, der ein Drehmomentbegrenzungsmittel besitzt, das zwischen einem Adapterelement mit Handgriff und einem Aufnahmeelement zur Aufnahme eines Werkzeugs angeordnet ist. Das Drehmomentbegrenzungsmittel ist dabei (Fig. 9) durch zwei Gleitflächenelemente mit einem durch diese steckbaren Abscherstift gebildet, von denen sich eine Gleitfläche an dem Adapterelement und die andere an dem Aufnahmeelement befindet und diese miteinander koaxial geführt sind. Sobald nun zwischen dem Handgriff und dem Werkzeug ein überhöhtes Drehmoment auftritt, welches dem Werkzeug schaden würde, wird der Abscherstift durch ein sich Verdrehen der Gleitflächenelemente abgeschert und dadurch das Werkzeug entlastet.

Ein Drehmoment zu begrenzen hat zweierlei Gründe. Zum einen dient es dazu, dass die Gewissheit besteht, dass z. B. eine Schraubverbindung mit einem definierten Drehmoment fixiert ist. Insbesondere im Automobilbereich oder im Kernkraftwerkbereich, bei dem es notwendig ist, Schrauben mit hohen Kräften unter sicherheitsrelevanten Aspekten festzuziehen, ist eine solche Vorrichtung notwendig. Sie dient dazu, dem Benutzer anzuzeigen, zu welchem Zeitpunkt tatsächlich der entsprechende Betrag des notwendigen Drehmoments erreicht ist, damit die Schraubverbindung fachgerecht ausgebildet ist.

Zum anderen dient das Drehmomentbegrenzungsmittel dazu, eine Schraube vor einem Überdrehen und damit einem Abreissen und dem damit verbundenen Verlust der Funktion zu schützen.

Das Drehmomentbegrenzungsmittel selbst ist bei einigen bekannten Ausführungsbeispielen als so genannte Rutschkupplung ausgeführt. Dies bedeutet, dass bei einem Aufbringen von höheren Drehmomenten die Kupplung aktiv wird und die aufgebrachte Kraft nicht mehr an das Aufnahmeelement weitergegeben wird. Ein Rastmechanismus entkoppelt das Adapterelement von dem Aufnahmeelement.

Eine solche Rutschkupplung ist beispielsweise in der DE 3149561 A1 (Heinlein, Hans (DE)) 15.12.1981 beschrieben, und zwar ist dort ein Drehmomentschlüssel zum Übertragen eines voreingestellten Drehmoments auf ein schraubbares Werkstück gezeigt, bei dem durch ein Verstellen des axialen Abstandes zwischen Reibscheiben das jeweils zu übertragende Drehmoment einstellbar ist. Zum Justieren der Reibscheiben werden hierbei mehrere so genannten Abscher-Justierstäbchen durch diese gesteckt, durch welche dann ein jeweiliges maximales Drehmoment bestimmt werden kann.

Ferner ist aus der US 4894043 (NIXON JR ROBERT E (US)) 16.01.1990 ist ein Drehmomentschlüssel bekannt, der aus einem Adapterelement und einem Aufnahmeelement sowie einem zwischen diesen angeordneten Drehmomentbegrenzungsmittel besteht. Dieses Drehmomentbegrenzungsmittel zeichnet sich dadurch aus, dass es aus zwei axial angeordneten Scheiben gebildet ist, die über ein gemeinsames Lager miteinander verbunden sind.

Zusätzlich ist an dem Adapterelement ein Schermittel vorgesehen, das zur Abscherung eines auf der radialen Aussenfläche des Drehmomentbegrenzungsmittels angeordneten Pins vorgesehen ist. Der Pin bzw. dessen Materialausgestaltung gibt vor, welches Drehmoment maximal von dem Adapterelement auf das Aufnahmeelement übertragen werden kann. Ist dieses maximale Drehmoment nicht erreicht, so wirkt der Pin als Mitnehmer, sodass das Adapterelement und das Aufnahmeelement schraubendreherartig bewegt werden kann. Ist das Drehmoment erreicht, so wird die Abscherplatte den entsprechenden Pin abtrennen, sodass keine weitere Mitnahme des Adapterelements bzw. Aufnahmeelements erfolgt. Das Adapterelement kann beliebig verdreht werden, ohne dass das Aufnahmeelement weiter verdreht wird. Um eine erneute Drehbegrenzung einstellen zu können, muss die Abscherplatte entfernt werden und ein neuer Pin in die speziell vorgesehene Bohrung eingesetzt werden. Der dargestellte Drehmomentschlüssel besteht aus einer Vielzahl von Bauteilen, die nicht dazu geeignet sind, insbesondere im medizinischen Bereich angewendet zu werden, da ein Sterilisierungsprozess sehr aufwendig ist.

Ferner ist aus der US 7335208 (REMICAVAGNA,PLOEMEUR(FR); DENISHUTEN,VINCENNES(FR); HUGUESMALANDAIN,SOUTHAVEN,TN(US) 26.02.2008 ein Drehmomentschlüssel bekannt, der aus einem griffartig ausgebildeten Adapterelement sowie einem Aufnahmeelement, das an seinem freien Ende schraubendreherartig ausgebildet ist, besteht. Dazwischen geschaltet, d. h zwischen dem Adapterelement und dem Aufnahmeelement, ist ein Drehbegrenzungsmittel vorgesehen. Dieses Drehbegrenzungsmittel zeichnet sich dadurch aus, dass ein stiftartiges Element über das Adapterelement eingeführt wird und an einer bestimmten Position gehalten wird. Ein Kugeldruckelement trägt dafür Sorge, dass der Stift positionsgerecht gehalten wird. Ferner sind Abschermittel vorhanden, die bei einem entsprechenden Drehmoment den Stift abscheren, sodass ein auf das Adapterelement aufgebrachte Drehmoment nicht mehr auf das Aufnahmeelement übertragen wird. Die Abscherung des Stiftes ist sowohl radial als auch in longitudinaler Richtung vorgesehen.

Dieser Drehmomentschlüssel ist für den medizinischen Bereich vorgeschlagen, insbesondere zur Anbringung von Knochenschrauben. Um zu verhindern, dass der Stift aus der entsprechenden Vorrichtung im Adapterelement wieder heraus fällt, ist ein federartiger Verschliessmechanismus vorgesehen.

Sobald eine Abscherung erfolgt ist, fällt ein Teil des Stiftes in ein Reservoir. Der übrige Teil des Stiftes muss dann von Hand entfernt werden, bevor ein neuer Stift eingeführt werden kann.

Ferner zeigt die EP 1880802 A2 (TELEFLEX MEDICAL INCORPORATED LIMERICK (US)) 14.03.2007 eine Vorrichtung mit einer Drehmomentbegrenzung mittels Abscherung, bei der mehreren koaxial hintereinander über Sollbruchstellen miteinander verbundene und axial federbelastete Abscherstifte vorgehen sind, die nach jeder Abscherung wie ein Magazin aufgrund der Federbelastung nachgeschoben werden können.

Schliesslich ist aus der US 7296500 B1 (MARTINELLI (US) 20.11.2007 eine weitere Variante eines Drehmomentsschlüssels für eine Anwendung im medizinischen Bereich bekannt, bei der über ein Zahnräderwerk dessen übertragendes Drehmoment mittels eines Abscherstiftes begrenzt wird.

### Nachtelle des Standes der Technik

Bei Anwendungen im medizinischen Bereich ist es notwendig, die vorgenannten Vorrichtungen vor deren Einsatz zu reinigen. Insbesondere das Einlegen der jeweiligen Vorrichtungen in Reinigungsmitteln sowie das Einbringen in Wascheinrichtungen können dazu führen, dass die Konstanz der z. B. in den Drehmomentbegrenzungsmitteln vorgesehenen Federn nachlässt. Da dabei die einzelnen Teile einer Vorrichtung auch entfettet werden, sollten möglichst wenig drehbare bzw. gleitende Teile an dieser vorhanden sein.

Bei der Anwendung von Drehmomentbegrenzungsschlüsseln ist es ferner notwendig, dass diese in regelmässigen Abständen neu geeicht werden müssen. Insbesondere im medizinischen Bereich ist dies extrem wichtig, da hier sehr hohe Genauigkeiten gefordert werden. Diese Eichvorgänge sind jedoch sehr aufwendig und kostenintensiv. Zudem sind die Werkzeuge in der Zeit, in denen die Drehmomentbegrenzungsschlüssel geeicht und gewartet werden, nicht verfügbar.

Ein weiterer wesentlicher Nachteil der vorgenannten Ausführungsformen von Drehmomentbegrenzungsschlüsseln ist, dass diese sehr komplex aufgebaut sind und daher nur kostenintensiv herstellbar sind.

Insbesondere bei den aus dem Stand der Technik bekannten Drehmomentschlüsseln, bei denen pinartige oder stiftartige Elemente zur Kopplung und Übertragung des Drehmoments von einem Adapterelement zu einem Aufnahmeelement eingesetzt werden, ergibt sich der Nachteil, dass keine definierte Position vorhanden ist, wobei der entsprechende Pin ohne Einblick auf die entsprechende Bohrung eingeführt werden muss. Dadurch muss durch eine Vielzahl von Versuchen probiert werden, bis der Pin tatsächlich die zwei übereinander liegenden Bohrungen trifft.

### Aufgabe der Erfindung

Aufgabe der Erfindung ist es daher, einen Drehmomentbegrenzungsschlüssel bereitzustellen, der insbesondere für den medizinischen Bereich geeignet ist und der die vorgenannten Nachteile des Standes der Technik nicht aufweist.

### Lösung der Aufgabe

Die Lösung der Aufgabe wird durch den kennzeichnenden Teil von Anspruch 1 gelöst.

### Vorteile der Erfindung

Durch die klare Trennung von Aufnahmeelement und Adapterelement mit dem dazwischen geschalteten Drehbegrenzungsmittel ist ein einfaches und kostengünstiges Hilfsmittel geschaffen worden. Das Drehbegrenzungsmittel besteht nicht wie es an sich aus dem Stand der Technik bekannt ist, z. B. aus einem komplexen federbelasteten Mechanismus, sondern ausschliesslich aus einem einzigen Bauteil.

Einer der wesentlichen Vorteile der Erfindung besteht auch darin, dass es bei der erfindungsgemässen Vorrichtung nicht erforderlich ist, diese zu kalibrieren, sobald sie einen entsprechenden Autoklaven verlassen hat. Aufgrund der Auswahl von Abscherstiften ist es ausschliesslich ein einfach gebautes und nicht mit komplexen Elementen versehenes Werkzeug, bei dem das maximale Drehmoment durch die Abscherstifte selbst bereitgestellt wird.

Vorzugsweise besteht der Abscherstift im Wesentlichen aus zwei Teilen, nämlich einem Halteteil und einem Pin, wobei beide Teile einstückig miteinander verbunden sind. Das Halteteil selbst ist in seinem Durchmesser grösser ausgestaltet als der Pin selbst, so dass aus technischer Sicht die Möglichkeit besteht, an dem Adapterelement ein Aufnahmemittel anzuordnen, das die Aufnahme des Halteteils des Abscherstiftes vorsieht und der Abscherstift derart angeordnet ist, dass sich der Pin in Richtung des Aufnahmemittels erstreckt. Auch im Bereich des Aufnahmemittels kann ein Aufnahmeteil vorgesehen sein, mit dem der Pin des Abscherstiftes zusammenwirkt. Aufgrund dieser form- und kraftschlüssigen Verbindung ist ein Aufbringen eines Drehmoments, das an dem Adapterelement angesetzt wird, in Bezug auf das Aufnahmemittel möglich.

Unmittelbar am Übergang des Abscherstiftes vom Halteteil zu dem Pin ist eine so genannte Sollbruchstelle vorgesehen. Sie ist derart ausgelegt, dass sie dem maximal aufzubringenden Drehmoment entspricht.

Daher nimmt die erfindungsgemässe Vorrichtung im Wesentlichen zwei Stellungen ein. Die erste Stellung ist dadurch gekennzeichnet, dass Drehmomente aufgebracht werden, die unterhalb des maximal aufzubringenden Drehmoments liegen. Dies bedeutet, dass das Adapterelement, gekoppelt über den Abscherstift mit dem Aufnahmeelement, eine entsprechende Drehbewegung der Schraube beziehungsweise Mutter ausübt. Ist die Schraube jedoch bereits fest, so steigt das aufzubringende Drehmoment an, bis zu dem Zeitpunkt, an dem das maximale Drehmoment überschritten wird. Ist das maximale Drehmoment überschritten, so schert der Pin vom Halteteil ab, in der Form, dass zunächst ein Fliessprozess in dem Bereich der Sollbruchstelle entsteht, bis dann ein vollständiges Abreissen erfolgt. Nach vollständigem Abreissen des Halteteils von dem Pin ist das Aufnahmeelement vom Adapterelement entkoppelt, so dass eine weitere Drehbewegung des Aufnahmeelements nicht mehr erfolgen kann, obwohl das Adapterelement an sich weitergedreht wird.

Ferner ist es zweckmässig, am Übergangsbereich vom Adapterelement zum Aufnahmeelement eine Kapsel vorgesehen, die zum einen die Funktion hat, das Adapterelement und das Aufnahmeelement derart zueinander zu fixieren, dass eine Drehbewegung möglich ist. Zudem hat die Kapsel die Eigenschaft, dass die abgetrennten Pins des Abscherstiftes aufgefangen werden und nicht in eine Wunde fallen können.

Weiterhin hat die Kapsel die Eigenschaft, unterschiedliche Aufnahmeelemente und Adapterelemente aufzunehmen, so dass eine beliebige Kombination je nach Bedarf möglich ist.

Dies bedeutet, dass beispielsweise mit einem Handgriff (Adapterelement) unterschiedliche Aufnahmeelemente, wie beispielsweise Kreuzschlitz-, Torx-, Schlitz- oder auch Imbusdreher, verbunden werden können.

Um eine einfache Zuordnung der Abscherstifte in Bezug auf das maximal aufzubringende Drehmoment zu ermöglichen, können unterschiedliche Ausgestaltungen der Abscherstifte vorgesehen sein. Zum einen ist es vorgesehen, die Abscherstifte farbig zu gestalten und hier dem entsprechend maximalen Drehmoment eine bestimmte Farbe zuzuordnen. Eine Alternativausführung kann darin bestehen, die Abscherstifte unterschiedlich in ihrer körperlichen Ausgestaltung auszubilden. Dies bedeutet, dass Abscherstifte, die beispielsweise ein kleines maximales Drehmoment besitzen, dass die rund ausgestaltet sind, wohingegen Abscherstifte, die höhere maximale Drehmomente übertragen, eckig oder vieleckig ausgebildet sind.

Es ist auch möglich, dass die zuvor beschriebene erfindungsgemässe Vorrichtung ein Magazin aufweisen kann, in dem entsprechende Abscherstifte vorgesehen sind. So ist es möglich, mehrere Schrauben, Muttern oder dgl. hintereinander mit einem entsprechenden Drehmoment anzuziehen, ohne neue Abscherstifte "nachzuladen".

Vor allem ist es fester Bestandteil der vorliegenden Erfindung, dass an dem Adapterelement ein Stoppstift fest angeordnet ist, der innerhalb einer vorgegebenen Kulisse, beispielsweise mit einem Segment von180 oder nur 45 Grad , hervorgerufen durch eine einfache Drehung des Adapterelements, ausüben kann. Die Kulisse ist dabei derart ausgelegt, dass in einer ersten Stellung, diese der Anlage des Stoppstifts an einem Ende der Kulisse entspricht und in dieser Stellung der Abscherstift in die entsprechenden Ausnehmungen eingeführt werden kann. Dadurch ist es möglich, für den Anwender ohne genaue Lokalisierung der Bohrung den Pin funktionsgerecht einzuführen. Dies kann sogar "blind" geschehen. Wenn dann der Abscherstift abreisst, so dreht sich das Adapterelement frei, und zwar soweit, bis der Stoppstift das andere Ende der Kulisse erreicht hat. Der Benutzer erhält aber eine Rückkopplung, dass der Abscherstift durchtrennt ist und das maximale vorgegebene Drehmoment erreicht ist. Fühlbar wird dies, da kein direkter Widerstand, hervorgerufen durch die einzudrehende Schraube oder Mutter, vorliegt. Erreicht der Stoppstift das andere Ende der Kulisse, kann wieder ein Drehmoment, jedoch nun ohne Begrenzung, aufgebracht werden. Dies hat den erheblichen Vorteil, dass die Vorrichtung weiter benutzbar und einsetzbar ist.

Ähnlich verhält es sich beim Herausdrehen einer Schraube oder Mutter. Ist der Stoppstift in Ausgangsstellung, so kann eine Drehbewegung auch ohne Abscherstift zum Entfernen (entgegengesetzte Drehung für das Eindrehen von Schraube oder Mutter) ausgeführt werden.

Ein weiterer wesentlicher Vorteil der Erfindung ist es, dass keine Verschleissteile vorgesehen sind. Es wird bewusst auf eine Abscherfläche, Abschervorrichtung oder eine Abscherhilfe verzichtet, um zu jedem Zeitpunkt die gleiche definierte Grösse für ein maximal aufzubringendes Drehmoment bereitstellen zu können.

Es wird noch einmal deutlich hervorgehoben, dass einer der wesentlichen Vorteile der erfindungsgemässen Vorrichtung darin besteht, dass diese nur aus zwei generellen Bauteilen besteht. Diese Bauteile sind jederzeit, insbesondere für den Sterilisationsprozess, vollständig auseinander zu bauen und ohne einen entsprechenden Genauigkeitsverlust auch wieder zusammenfügbar. Bewegliche Elemente, die insbesondere bei Sterilisationsprozessen auch bei mehrfachen anderen Anwendungsprozessen sich verändern können, weist diese Vorrichtung nicht auf. Ein weiterer wesentlicher Vorteil der Erfindung ist es, dass auf sehr einfache Art und Weise die Pins zur Drehmomentbegrenzung eingeführt werden können, aber auch der Drehmomentschlüssel dann funktionsgerecht ist, wenn ein Pin bereits abgeschert ist und möglicherweise noch eine Schraube entsprechend nachgezogen werden muss.

Weitere vorteilhafte Ausgestaltungen gehen aus der nachfolgenden Beschreibung, den Ansprüchen sowie der Zeichnung hervor.

### Zeichnungen

Es zeigen:
- Fig. 1: eine schematische Prinzipdarstellung der erfindungsgemässen Vorrichtung im Schnitt;
- Fig. 2: eine perspektivische Darstellung der erfindungsgemässen Vorrichtung, bestehend aus einem Aufnahmeelement, einem Adapterelement und einem Drehmomentbeg renzungsmittel;
- Fig. 3: eine vergrösserte Darstellung des Adapterelements gemäss Fig. 2;
- Fig. 4: eine vergrösserte Darstellung des Aufnahmeelements gemäss Fig. 2;
- Fig. 5: eine perspektivische Ansicht auf ein Ausführungsbeispiel eines Abscherstiftes;
- Fig. 6: eine vergrösserte Darstellung einer Kapsel zur Verbindung zwischen Adapterelement und Aufnahmeelement;
- Fig. 7: eine Prinzipdarstellung des Drehbegrenzungsmittels zur Verdeutlichung der Funktion der erfindungsgemässen Vorrichtung;
- Fig. 8: eine schematische Darstellung der ersten Stellung der erfindungsgemässen Vorrichtung und
- Fig. 9: eine schematische Darstellung einer weiteren Stellung der erfindungsgemässen Vorrichtung.

### Beschreibung der Ausführungsbeispiele

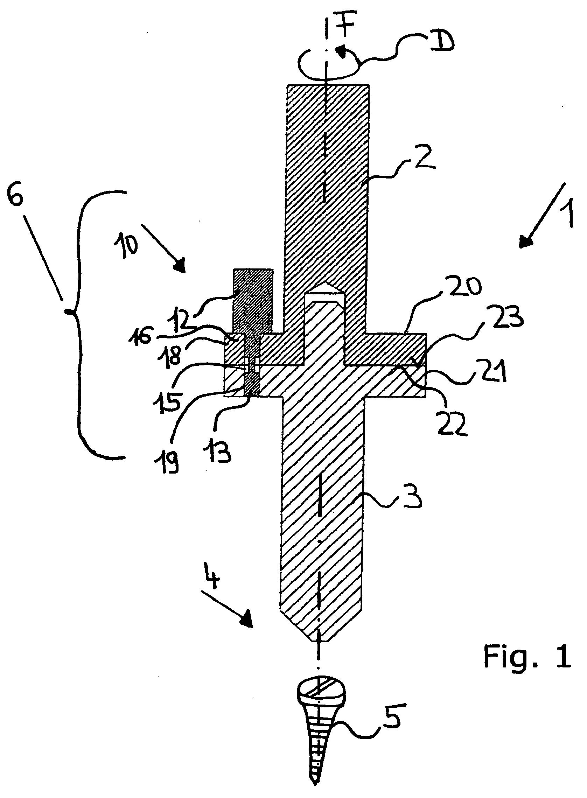

In Fig. 1 ist schematisch die erfindungsgemässe mit 1 bezeichnete Vorrichtung zum begrenzten Aufbringen eines Drehmoments dargestellt. Das Drehmoment, das entweder mittels einer Hand oder einer mechanischen, elektrischen, pneumatischen oder hydraulischen Vorrichtung aufgebracht wird, soll bis zu einer definierten Maximalgrösse auf eine Schraube oder eine Mutter übertragen werden.

Die Vorrichtung 1 besteht aus einem Adapterelement 2 und einem Aufnahmeelement 3, wobei an dem mit 4 bezeichneten freien Ende des Aufnahmeelements 3 eine Vorrichtung vorgesehen ist, mittels der eine Schraube 5 eindrehbar ist.

Die erfindungsgemässe Vorrichtung 1 ist derart ausgestaltet, dass ein über das Adapterelement 2 aufgebrachte Drehmoment D in der Ausbildung einer Drehkraft F über ein Drehmomentbegrenzungsmittel 6 auf das Aufnahmeelement 3 übertragen werden kann. Die beiden Elemente, nämlich das Adapterelement 2 und das Aufnahmeelement 3 sind stabförmig ausgebildet und bei dem hier dargestellten Prinzipbeispiel fluchtend zueinander ausgerichtet. Durch das Drehmomentbegrenzungsmittel 6 sind die beiden Elemente, nämlich das Adapterelement 2 und das Aufnahmeelement 3 voneinander entkoppelbar. Das Drehmomentbegrenzungsmittel 6 hat die Aufgabe, die beiden Elemente miteinander zu koppeln, derart, dass zwei Stellungen ausführbar sind. In einer ersten Stellung kann das auf das Adapterelement 2 aufgebrachte Drehmoment auf das über das Drehmomentbegrenzungsmittel 6 auf das Aufnahmeelement 3 übertragen werden, sofern das aufgebrachte Drehmoment D kleiner ist als das maximal zulässige Drehmoment. Die zweite Stellung sieht vor, dass das Drehmomentbegrenzungsmittel 6 aktiv wird, weil von dem Adapterelement 2 auf das Aufnahmeelement 3 übertragene Drehmoment grösser ist als das maximale Drehmoment.

Das Drehmomentbegrenzungsmittel 6 umfasst ein erstes und ein zweites Gleitflächenelement 20, 21, die dem Adapterelement 2 beziehungsweise dem Aufnahmeelement 3 zugeordnet und dort befestigt sind. Bei dem vorliegenden Beispiel bilden sie mit dem Adapterelement 2 beziehungsweise dem Aufnahmeelement 3 jeweils ein einstückiges Teil. Die Gleitflächenelemente 20, 21 sind scheibenartig ausgebildet und besitzen Gleitflächen 22, 23. Das erste oder das zweite Gleitflächenelement 20, 21 weist zusätzlich eine Kulisse 24 auf, die mit einem Stoppstift 25, der ortsfest an dem Adapterelement 2 zugeordneten Gleitflächenelement 20 oder ortsfest an dem Aufnahmeelement 3 zugeordneten Gleitflächenelement 21 angeordnet ist, zusammenwirkt.

Eine einfache, in den Zeichnungen nicht näher dargestellte Ausführungsform der erfindungsgemässen Vorrichtung 1 sieht vor, dass das Adapterelement 2 den Stoppstift 25 besitzt, der in die Kulisse 24, die Teil des Aufnahmeelements 3 ist, führbar ist. Hierfür hat die Kulisse 24 am Umfang des Gleitflächenelements 21 eine Bogenlänge, die einem freien Winkel von 45 Grad entspricht, und zwar innerhalb der sich der Stoppstift frei hin- und herbewegen kann. Bei einer Anlage des Stoppstiftes 25 innerhalb der Kulisse 24 an der Seite des Abscherstifts 10, d. h. wo dieser in entsprechende Ausnehmungen 16, 19 einführbar ist, kann sodann eine Kopplung zwischen dem Adapterelement 2 und dem Aufnahmeelement 3 vorgenommen werden, da sich dann die Ausnehmungen 16, 19 decken und der Abscherstift 10 in diese problemlos gesteckt werden kann. Wird durch ein überhöhtes Drehmoment der Abscherstift 10 abgetrennt, so kann das Adapterelement 2 noch soweit verdreht werden, ohne dass sich das Aufnahmeelement 3 weiter verdreht, bis der Stoppstift 25 die Kulisse 24 vollständig durchlaufen hat und an die gegenüberliegenden Seite der Kulisse 24 zur Anlage gelangt. Mit dem Erreichen des Stoppstifts 25 an dieser Anlage, sind das Adapterelement 2 und das Aufnahmeelement 3 wieder miteinander gekoppelt, und zwar ohne eine Drehmomentbegrenzung.

Die technische Wirkung des Drehmomentbegrenzungsmittels 6 wird derart erzielt, indem das Adapterelement 2 von dem Aufnahmeelement 3 entkoppelbar ist. Als erfindungswesentliches Merkmal wird zuerst der Abscherstift 10 eingesetzt, der eine kraft- und formschlüssige Verbindung zwischen dem Adapterelement 2 und dem Aufnahmeelement 3 herstellt. Dabei hat der Abscherstift 10 zwei Bereiche, nämlich einen Halteteil 12 und einen Pin 13, zwischen denen bei dem hier dargestellten Ausführungsbeispiel eine Sollbruchstelle 15 vorgesehen ist.

Das Halteteil 12 des Abscherstiftes 10 ist an einer in Bezug auf das Adapterelement 2 ortsfeste Halteeinrichtung 18 angeordnet bzw. gehalten, wobei für die Aufnahme des Pins 15, hierfür zuerst die erste Ausnehmung 16 und von dieser aus erstreckt sich der Pin 15 dann frei in die zweite Ausnehmung 19, die in Bezug auf das Aufnahmeelement 3 an diesem ortsfest ausgebildet ist. Durch die kraft- und formschlüssige Verbindung des Abscherstifts 10 wird das aufgebrachte Drehmoment D auf das freie Ende 4 des Aufnahmeelements 3 übertragen. Der Kraftfluss erstreckt sich somit von dem Adapterelement 2, über den Abscherstift 10 bis zum freien Ende 4 des Aufnahmeelements 3.

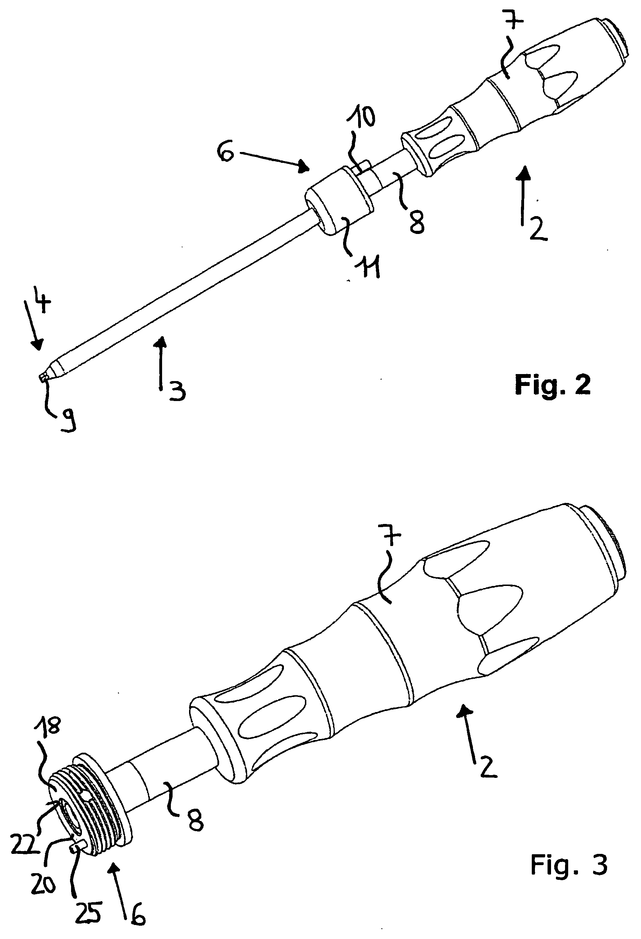

In den Fig. 2 bis 7 ist ein in der Praxis einsetzbares Ausführungsbeispiel der erfindungsgemässen Vorrichtung 1 dargestellt, und zwar mit einem Adapterelement 2, das einen Handgriff 7 besitzt, der in eine stabförmige Weiterbildung 8 übergeht, die in das Drehmomentbegrenzungsmittel 6 mündet. Von letzterem erstreckt sich wiederum koaxial das ebenfalls bei diesem Ausführungsbeispiel stabförmig ausgebildete Aufnahmeelement 3 bis zu seinem freien Ende 4, wobei an diesem ein Schraubenzieherteil 9 angeordnet ist, um beispielsweise eine Schraube 5, wie sie in Fig. 1 dargestellt ist, einzudrehen.

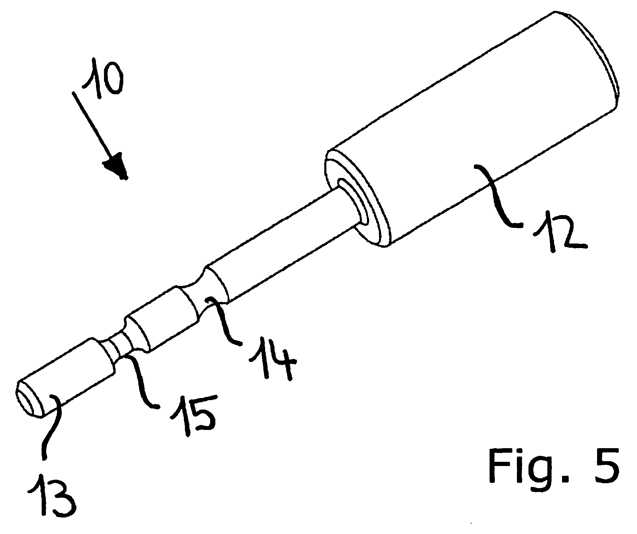

In Fig. 5 ist die Ausführungsform des Abscherstifts 10 dargestellt. Dieser besteht aus dem Halteteil 12, der Sollbruchstelle 15 sowie dem Pin 13. Eine zusätzliche Einkerbung 14 ist dafür vorgesehen, dass ein einmal in die Halteeinrichtung 18 eingeführter Abscherstift 10 nicht ohne weiteres (ohne zusätzliche Zugkräfte) wieder aus dieser herausfallen kann. Zu diesem Zweck ist innerhalb der Halteeinrichtung 18 eine federbelastete Druckkugel vorgesehen, die in die Einkerbung 14 eingreift, wenn der Abscherstift 10 korrekt positioniert ist.

Alternativ hierzu kann auch eine formgerechte Presspassung oder andere Mittel vorgesehen werden, die geeignet sind, den Abscherstift 10 in jeder Lage der Vorrichtung 1 zu halten.

Bei dem hier dargestellten Ausführungsbeispiel ist der Abscherstift 10 rotationssymmetrisch ausgebildet. Andere Querschnittsformen sind ebenfalls denkbar.

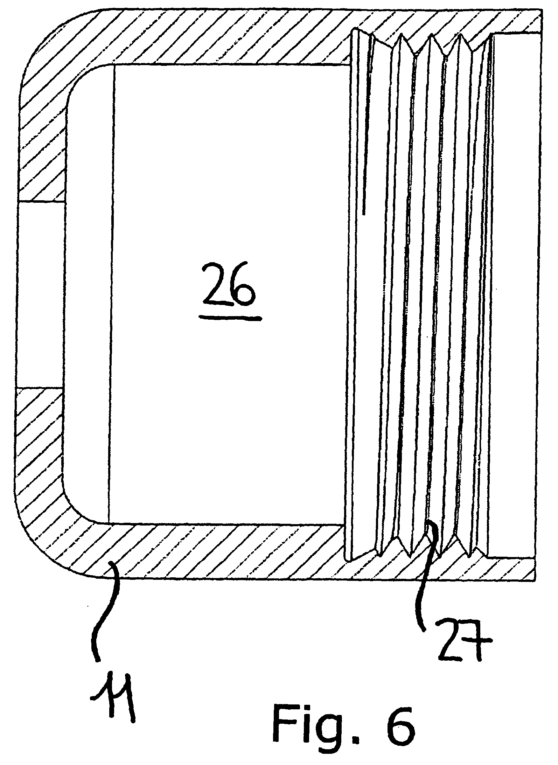

In Fig. 6 ist eine Kappe 11 dargestellt, die vorgesehen ist, um jeweils den abgescherten Teil des Abscherstifts 10, nämlich den Pin 13 aufzufangen und zu sammeln. Hierzu weist die Kappe 11 einen Hohlraum 26 auf, der derart bemessen ist, dass mehrere Pins 13 von diesem aufgenommen werden können. Die so gesammelten Pins 13 können wieder aufbereitet werden. Die Kappe 11 weist ferner ein Gewinde 27 auf, das dazu geeignet ist, die Kappe 11 ortsfest anzubringen, und zwar an dem Adapterelement 2. Hierfür ist an dem Adapterelement 2 an dessen Gleitflächenelement 20, ein zu dem Gewinde 27 korrespondierendes Gewinde 28 vorgesehen. Durch einfaches Schrauben kann die Kappe 11 angebracht und wieder entfernt werden. Alternativ können auch Bajonett- und Schnappverschlüsse vorgesehen werden.

Eine weitere in den Zeichnungen nicht näher dargestellte Ausbildungsform sieht vor, dass alternativ zu den bisher beschriebenen einzelnen Abscherstiften, eine Abscherstange eingeführt wird, die magazinartig mehrere Schraubvorgänge zulässt, bei der jeweils das entsprechende Drehmoment überschritten wird. Hierzu ist vorgesehen, dass die einzelnen Abscherstifte als eine Stange ausgebildet sind, die sukzessive nach dem jeweiligen Überschreiten des Drehmomentes wieder in die einzelnen Ausnehmungen 16 bzw. 19 hereinrutscht und so wieder eine Koppelung der beiden Teile, nämlich des Adapterelementes 2 und des Aufnahmeelements 3 herbeiführt. Dadurch ist es möglich, mit einer einzigen "Ladung" mehrere drehmomentbegrenzte Schraub- bzw. Drehvorgänge auszuüben. Eine weitere Alternative sieht vor, dass eine magazinartige Bevorratung der jeweiligen Abscherstifte vorgesehen ist. Diese rücken selbsttätig nach, sobald der Stoppstift 25 am Ende der Kulisse 24 anschlägt und wieder in seine Ursprungsposition verfahren wird, bei der die beiden Ausnehmungen 16, 19 fluchtend zueinander angeordnet sind.

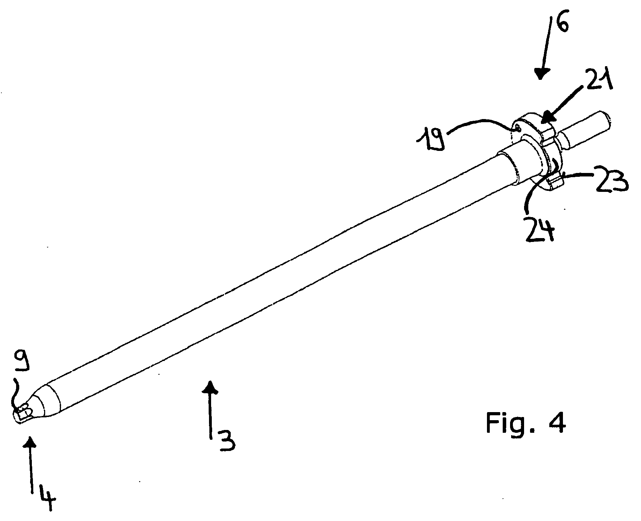

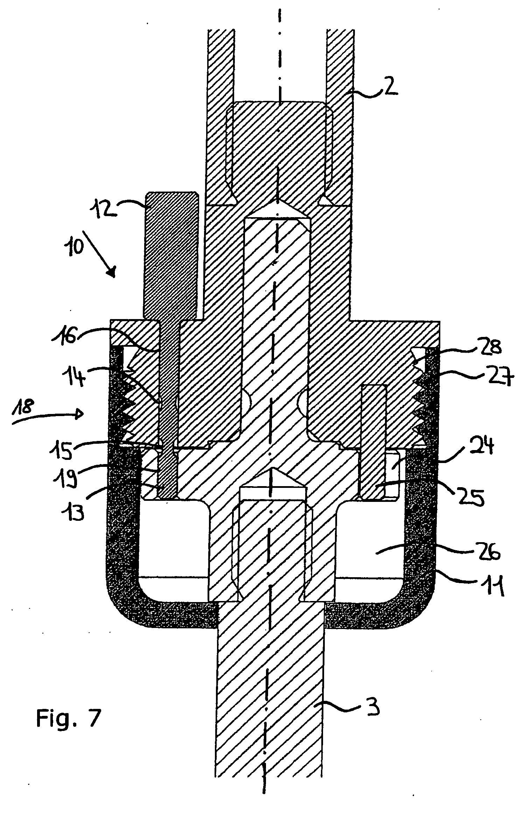

Kern der Erfindung und damit auch der Ausführungsbeispiele ist die Ausbildung des Drehmomentbegrenzungsmittels 6. Es ist ausführlich in Fig.7 dargestellt, wobei die Fig. 3 und 4 die Einzelausbildungen des Drehmomentbegrenzungsmittels 6 zeigen.

Um die Komplexität der Bauteile zur Herstellung einer solchen Vorrichtung gering zu halten, ist es vorteilhaft, wie auch in Fig. 7 dargestellt, das Adapterelement 2 zweigeteilt auszubilden. Als Endprodukt ist es jedoch ein einziges Teil. Dies gilt auch für das ähnlich aufgebaute Aufnahmeelement 3.

Es ist auch möglich, dass sich die Kulisse 24 über nahezu den gesamten Umfang des Drehmomentbegrenzungsmittels 6 erstreckt. Vorzugsweise nimmt jedoch die Kulisse 24 ein Winkelsegment von 90° ein.

Das Adapterelement 2 und das Aufnahmeelement 3 sind jeweils axial geführt und derart zueinander angeordnet, dass die Achsen von dem Adapterelement 2 und dem Aufnahmeelement 3 zueinander fluchtend sind, wobei im Bereich des Drehmomentbegrenzungsmittels 6 die Gleitflächen 22, 23 der Gleitflächenelemente 20, 21 aufeinander gleiten können.

### Funktionsweise

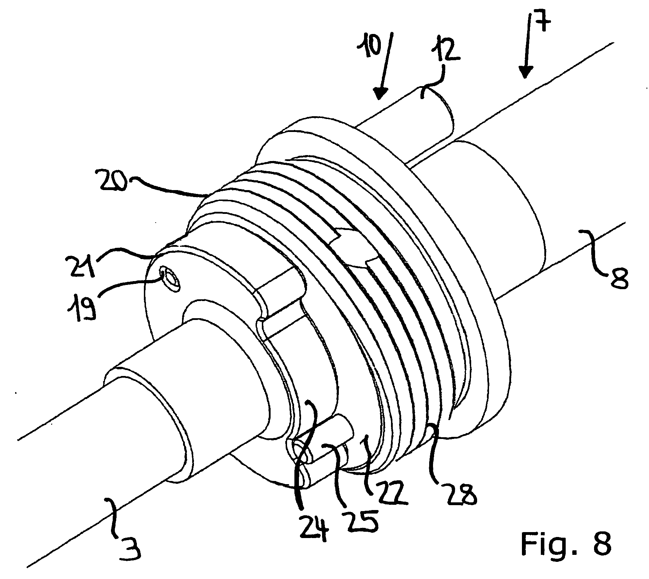

Die erste Stellung (Fig. 8) ist derart ausgebildet, dass der Pin 13 das Adapterelement 2 mit dem Aufnahmeelement 3 gekoppelt ist, so dass die auf das Adapterelement 2 aufgebrachte Kraft F unmittelbar auf das Aufnahmeelement 3 übertragen werden kann. Der Pin 13 ist in den Ausnehmungen 16 und 19 platziert und einstückig mit dem Halteteil 12 ausgebildet. Der Stoppstift 25 liegt an dem Ende der Kulisse 24 an, das dem Abscherstift 10 am nächsten ist. So ist es möglich, sehr einfach den Abscherstift 10 bzw. dessen Pin 13 in die Ausnehmungen 16 und 19 einzuführen, da diese in dieser Position miteinander fluchten.

Überschreitet die Kraft in der Ausführung eines Drehmoments eine maximale Grösse, so wird der Pin 13 im Bereich der Sollbruchstelle 15 abgeschert. In Fig. 9 ist dieser Vorgang dargestellt. Dies führt wiederum dazu, dass eine Entkoppelung stattfindet, da der übrige Teil des Pins 13 in die kapselartige Ausbildung 11, die Teil des Drehmomentbegrenzungsmittels 6 ist, bzw. in dessen Hohlraum 26 fällt. Ein Teil des Abscherstiftes 10, nämlich der Halteteil 12, verbleibt weiterhin in der Ausnehmung 16, die dem Adapterelement 2 zugeordnet ist. Dadurch wird verhindert, dass der Rest des Abscherstiftes 10 ungewollt von der erfindungsgemässen Vorrichtung 1 abfällt. Damit ist eine Entkoppelung eingetreten und die Kraft kann nicht mehr von dem Adapterelement 2 mittels des Abscherstiftes 10 auf das Aufnahmeelement 3 übertragen werden. Das Adapterelement 2 gleitet nun auf den Gleitflächen 22, 23 und der Stoppstift 25 kann entlang der Kulisse 24 geführt werden und zwar soweit, bis dieser an dem anderen Ende der Kulisse 24 zur Anlage kommt. Sobald dieser Zustand erreicht wird, kann ohne Drehmomentbegrenzungsmittel 6 weiter die Schraube 5 eingedreht werden.

Die erfindungsgemässe Vorrichtung 1 kann aus unterschiedlichen Ausbildungen von Adapterelementen 2 in Form von Handgriffen 7 sowie von Ausbildungen im Bereich des Aufnahmeelements 3 bestehen. Zusätzlich sind als drittes Bauteil Drehmomentbegrenzungsmittel 6 vorgesehen, die mit entsprechenden Abscherstiften 10, die beispielsweise farblich oder über die Form gekennzeichnet sind, bestückt werden können, um die Grenze des maximalen Drehmoments festzulegen. Alle Bauteile können in einem Set angeboten werden.

### Bezugszeichenlist

- 1: Vorrichtung
- 2: Adapterelement
- 3: Aufnahmeelement
- 4: freies Ende
- 5: Schraube
- 6: Drehmomentbegrenzungsmittel
- 7: Handgriff
- 8: stabförmige Weiterbildung
- 9: Schraubendreher
- 10: Abscherstift
- 11: Kappe
- 12: Halteteil
- 13: Pin
- 14: Einkerbung
- 15: Sollbruchstelle
- 16: Ausnehmung
- 17: -
- 18: Halteeinrichtung
- 19: Ausnehmung
- 20: Gleitflächenelement
- 21: Gleitflächenelement
- 22: Gleitfläche
- 23: Gleitfläche
- 24: Kulisse
- 25: Stoppstift
- 26: Hohlraum Kappe 11
- 27: Gewinde Kappe 11
- 28: Gewinde
- D: Drehmoment
- F: Drehkraft

## Patentansprüche

1. Vorrichtung (1) zum Eindrehen von Schrauben (5), insbesondere von im medizinischen Bereich verwendeten Knochenschrauben, die ein Adapterelement (2) zur Aufnahme eines Handgriffs (7) oder eines Hilfswerkzeugs und ein Aufnahmeelement (3) zur Aufnahme für die Schrauben (5) aufweist, während das Adapterelement (2) und das Aufnahmeelement (3) über ein Drehmomentbegrezungsmittel (6) mit einem Abscherstift (10) zum Begrenzen eines beim Eindrehen derSchrauben (5) aufzubringendes maximal zulässiges Drehmoments miteinander koppelbar sind sowie über ein erstes und ein zweites jeweils scheibenförmig ausgebildetes und mit jeweils einer Gleitfläche (22; 23) versehenes Gleitflächenelement (20; 21) koaxial zueinander führbar sind, und zwar indem jedes der Gleitflächenelemente (20; 21) eine Ausnehmung (16; 19) für die gemeinsame Aufnahme des Abscherstiftes (10) aufweiset, wobei das erste Gleitflächenelement (20) dem Adapterelement (2) und das zweite Gleitflächenelement (21) dem Aufhahmeelement (3) zugeordnet ist,
**dadurch, gekennzeichnet, dass**
das zweite Gleitflächenelement (21) zusätzlich eine Kulisse (24) besitzt, die mit einem an dem ersten Gleitflächenelement (20) ortsfest angeordneten Stoppstift (25) derart zusammenwirkt, dass in einer ersten Stellung das Adapterelement (2) mit dem Aufnahmeelement (3) über den Abscherstift (10) koppelbar ist und hierfür die Ausnehmungen (16; 19) miteinander fluchten, wobei der Stoppstift (25) innerhalb der Kulisse (24) an dem einem Ende der Kulisse anliegt, wogegen nach Erreichen des maximalen Drehmoments und einem Abscheren des Abscherstiftes (10), der Stoppstift (25) innerhalb der Kulisse (24) gleitet bis dieser an dem anderen Ende der Kulisse (24) zur Anlage kommt und das Adapterelement (2) mit dem Aufnahmeelement (3) derart gekoppelt ist, dass keine Drehmomentbegrenzung erfolgt..

2. Vorrichtung nach Anspruch 1, **dadurch gekennzeichnet, dass** für die Kulisse (24) des Gleitflächenelements (21) ein Winkelsegment von mindestens 45 ° vorgesehen ist.

3. Vorrichtung nach Anspruch 1 oder 2, **dadurch gekennzeichnet, dass** der Stoppstift (25) radial ausserhalb der Längsachse der Vorrichtung (1) an dem jeweiligen Gleitflächenelement (20) angeordnet ist.

4. Vorrichtung nach mindestens einem der vorhergehenden Ansprüche, **dadurch gekennzeichnet, dass** der Abscherstift (10) aus einem von dem ersten Gleitflächenelement (20) gehaltenen Halteteil (12) und einem in die Ausnehmungen (16;19) steckbaren Pin (13) besteht.

5. Vorrichtung nach Anspruch 4, **dadurch gekennzeichnet, dass** der Abscherstift (10) im Bereich des Übergangs von der Ausnehmung (16) des ersten Gleitflächenelements (20) zur Ausnehmung (19) des zweiten Gleitflächenelements (21) eine Sollbruchstelle (15) aufweist.

6. Vorrichtung nach Anspruch 5, **dadurch gekennzeichnet, dass** das Material der Sollbruchstelle (15) Massgabe für das maximal aufzubringende Drehmoment ist.

7. Vorrichtung nach einem der vorhergehenden Ansprüche, **dadurch gekennzeichnet, dass** das Drehmomentbegrenzungsmittel (6) von einer Kapsel (11) zum Auffangen von abgescherten Pins (13) umgeben ist.

## Claims

1. Device (1) for turning in screws (5), in particular bone screws used in the medical field, which device (1) has an adapter element (2) for receiving a handle (7) or an auxiliary tool, and a receiving element (3) for receiving the screws (5), while the adapter element (2) and the receiving element (3) can be coupled to each other via a torque limiter (6) with a shear pin (10) for limiting a maximum permissible torque to be applied when turning in the screws (5) and can be guided coaxially to each other via a first and a second disc-shaped slide-surface element (20; 21), each provided with a slide surface (22; 23), and specifically by each of the slide-surface elements (20; 21) having a recess (16; 19) for jointly receiving the shear pin (10), wherein the first slide-surface element (20) is assigned to the adapter element (2) and the second slide-surface element (21) is assigned to the receiving element (3),
**characterized in that**
the second slide- surface element (21) additionally has a slotted guide (24) which interacts with a stop pin (25), arranged stationary on the first slide-surface element (20), in such a way that, in a first position, the adapter element (2) can be coupled to the receiving element (3) via the shear pin (10), for which purpose the recesses (16; 19) are flush with each other, wherein the stop pin (25) inside the slotted guide (24) bears on one end of the slotted guide, whereas, after the maximum torque is reached and the shear pin (10) shears off, the stop pin (25) inside the slotted guide (24) slides until it comes to bear on the other end of the slotted guide (24) and the adapter element (2) is coupled to the receiving element (3) in such a way that there is no torque limitation.

2. Device according to Claim 1, **characterized in that** an angle segment of at least 45° is provided for the slotted guide (24) of the slide-surface element (21).

3. Device according to Claim 1 or 2, **characterized in that** the stop pin (25) is arranged radially outside the longitudinal axis of the device (1) on the respective slide-surface element (20).

4. Device according to at least one of the preceding claims, **characterized in that** the shear pin (10) consists of a holding part (12) held by the first slide-surface element (20), and of a tip (13) that can be inserted into the recesses (16; 19).

5. Device according to Claim 4, **characterized in that** the shear pin (10) has a predetermined breaking point (15) in the area of the transition from the recess (16) of the first slide-surface element (20) to the recess (19) of the second slide- surface element (21).

6. Device according to Claim 5, **characterized in that** the material of the predetermined breaking point (15) is critical in determining the maximum torque that can be applied.

7. Device according to one of the preceding claims, **characterized in that** the torque limiter (6) is surrounded by a capsule (11) for catching shorn- off tips (13).

## Revendications

1. Dispositif (1) pour visser des vis (5), en particulier des vis à os utilisées dans le domaine médical, lequel dispositif présente un élément adaptateur (2) destiné à recevoir une poignée (7) ou un outil auxiliaire et un élément de réception (3) destiné à recevoir les vis (5), tandis que l'élément adaptateur (2) et l'élément de réception (3) peuvent être accouplés l'un à l'autre par le biais d'un moyen de limitation de couple (6) avec une goupille de cisaillement (10) pour limiter un couple maximal admissible devant être appliqué lors du vissage des vis (5), et peuvent être guidés coaxialement l'un par rapport à l'autre par le biais d'un premier et d'un deuxième élément à surface de glissement (20 ; 21) réalisés à chaque fois sous forme de disque et pourvus d'une surface de glissement respective (22 ; 23), et ce par le fait que chacun des éléments à surface de glissement (20 ; 21) présente un évidement (16 ; 19) pour recevoir en commun la goupille de cisaillement (10), le premier élément à surface de glissement (20) étant associé à l'élément adaptateur (2) et le deuxième élément à surface de glissement (21) étant associé à l'élément de réception (3),
**caractérisé en ce que**
le deuxième élément à surface de glissement (21) possède en outre une coulisse (24) qui coopère avec une goupille d'arrêt (25) disposée fixement sur le premier élément à surface de glissement (20) de telle sorte que dans une première position, l'élément adaptateur (2) puisse être accouplé à l'élément de réception (3) par le biais de la goupille de cisaillement (10) et que pour cela les évidements (16 ; 19) soient en affleurement l'un avec l'autre, la goupille d'arrêt (25) s'appliquant à l'intérieur de la coulisse (24) sur l'une des extrémités de la coulisse, tandis qu'après l'obtention du couple maximal et cisaillement de la goupille de cisaillement (10), la goupille d'arrêt (25) glisse à l'intérieur de la coulisse (24) jusqu'à ce que celle-ci vienne en appui contre l'autre extrémité de la coulisse (24) et l'élément adaptateur (2) est accouplé avec l'élément de réception (3) de telle sorte qu'il ne se produise aucune limitation de couple.

2. Dispositif selon la revendication 1, **caractérisé en ce que** pour la coulisse (24) de l'élément à surface de glissement (21), il est prévu un segment coudé d'au moins 45°.

3. Dispositif selon la revendication 1 ou 2, **caractérisé en ce que** la goupille d'arrêt (25) est disposée radialement à l'extérieur de l'axe longitudinal du dispositif (1) au niveau de l'élément à surface de glissement respectif (20).

4. Dispositif selon au moins l'une quelconque des revendications précédentes, **caractérisé en ce que** la goupille de cisaillement (10) se compose d'une partie de retenue (12) retenue par le premier élément à surface de glissement (20) et d'une broche (13) pouvant être enfichée dans les évidements (16 ; 19).

5. Dispositif selon la revendication 4, **caractérisé en ce que** la goupille de cisaillement (10) présente, dans la région de la transition de l'évidement (16) du premier élément à surface de glissement (20) à l'évidement (19) du deuxième élément à surface de glissement (21), une zone destinée à la rupture (15).

6. Dispositif selon la revendication 5, **caractérisé en ce que** le matériau de la zone destinée à la rupture (15) est déterminant pour le couple maximal à appliquer.

7. Dispositif selon l'une quelconque des revendications précédentes, **caractérisé en ce que** le moyen de limitation de couple (6) est entouré par une capsule (11) pour collecter des broches cisaillées (13).
